# EUROPEAN PATENT APPLICATION

(11) **EP 3 553 051 A1**
(43) Date of publication of application: **16.10.2019**
(21) Application number: 17877424.6
(22) Date of filing: 30.11.2017
(51) Int. Cl.: C07D 213/74, C07D 471/04, C07B 61/00

(54) **METHOD FOR PRODUCING 3-(PYRIDYL-2-AMINO)PROPIONITRILE AND ANALOGUES THEREOF**

(30) Priority: 06.12.2016 JP 2016236409
(71) Applicant: Nippon Kayaku Kabushiki Kaisha, Tokyo 100-0005 (JP)
(72) Inventor: KAMO Tomohiro, Kamisu-shi Ibaraki 314-0255 (JP); ATARASHI Taiki, Kamisu-shi Ibaraki 314-0255 (JP); KURODA Hirokazu, Kamisu-shi Ibaraki 314-0255 (JP); KOBAYASHI Takeru, Kamisu-shi Ibaraki 314-0255 (JP)
(74) Representative: Gille Hrabal
(86) International application number: PCT/JP2017/043095
(87) International publication number: WO 2018/105492

(57) **Abstract**

A method for producing a compound represented by formula (1), the method comprising:
- mixing a compound represented by formula (2) and a compound represented by formula (3) in the presence of a base to react the two.

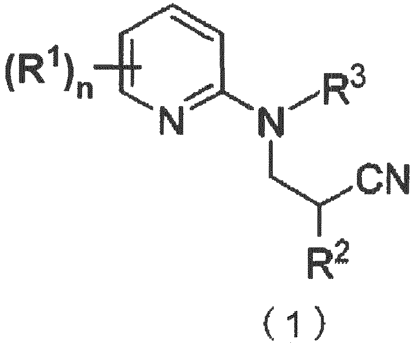

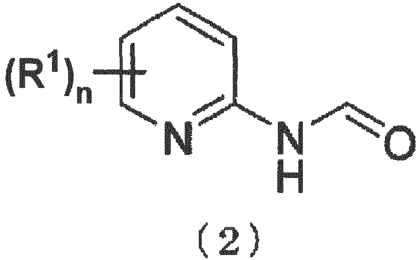

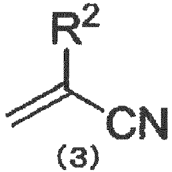

## Description

### Technical Field

The present invention relates to, for example, a method for producing 3-(pyridyl-2-amino)propionitrile and its analogues used as synthetic intermediates of medicines, pesticides, and the like.

### Background Art

In References 1 and 2, mesoionic compounds are known to exhibit an insecticidal effect and an excellent pest control effect against many insect pests. In Reference 1, a mesoionic compound having a cyanoethyl group as a substituent group is described. 3-(Pyridyl-2-amino)propionitrile and its analogues correspond to synthetic intermediates of mesoionic compounds described in Reference 1, and specific synthesis methods thereof are described in Patent Documents 1 and 3 and Non-Patent Documents 1 and 2. However, its synthesis method has many problems such as many steps, low yield, and limited reaction of substituent groups.

### Prior Art Documents

### Patent Document

**Patent Document 1:** WO2015/104822
**Patent Document 2:** JP 2013-501061A
**Patent Document 3:** JP 2001-519351A

### Non-Patent Document

**Non-Patent Document 1:** Bulletin de la Societe Chimique de France (1957), 718-721, 721-723.
**Non-Patent Document 2:** Chimica Therapeutica (1973), 8 (2), 239-241.

### Summary of Invention

### Problem to be Solved by the Invention

The present invention relates to provide a method for efficiently producing 3-(pyridyl-2-amino)propionitrile and its analogues.

### Means to Solve the Problem

The present inventors have performed intensive investigations on a method for producing 3-(pyridyl-2-amino)propionitrile and its analogues, and as a result have found that it solves the above described problems and have reached the present invention. The present invention also includes methods for producing 3-(pyridyl-2-amino)propionitrile and an important intermediate of its analogues, N-(2-cyanoethyl)-N-(2-pyridyl)formamide.

The present invention is as follows.

### [Invention 1]

A method for producing a compound represented by formula (1): 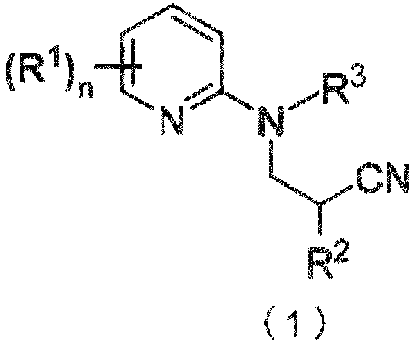
wherein each R¹ independently represents a hydrogen atom, a halogen atom, a cyano group, a C1 to C4 alkyl group, a C1 to C4 haloalkyl group, a C1 to C4 alkoxy group, a C1 to C4 haloalkoxy group, a C1 to C4 alkylthio group, a C1 to C4 haloalkylthio group, a C1 to C4 alkylsulfonyl group, a C1 to C4 haloalkylsulfonyl group, a C1 to C4 alkylsulfone group, a C1 to C4 haloalkylsulfone group, or a pentafluorosulfanyl group;
R² is a hydrogen atom, a C1 to C4 alkyl group, a C1 to C4 haloalkyl group, or a C1 to C4 alkoxy group;
R³ is a hydrogen atom or a formyl group; and
n is 0 to 4,
the method comprising:
- mixing a compound represented by formula (2): 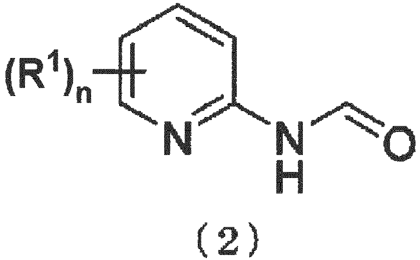 wherein each R¹ is as defined in formula (1), and
   a compound represented by formula (3): 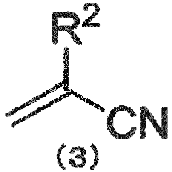 wherein R² is as defined in formula (1),
   in the presence of a base to react the two.

### [Invention 2]

The method according to Invention 1, wherein each R¹ independently represents a hydrogen atom, a halogen atom, a C1 to C4 alkyl group, a C1 to C4 haloalkyl group, or a C1 to C4 haloalkoxy group; and
R² is a hydrogen atom or a C1 to C4 alkyl group.

### [Invention 3]

The method according to Invention 1 or 2, wherein the base is one or more selected from lithium carbonate, sodium carbonate, potassium carbonate, cesium carbonate, lithium hydroxide, sodium hydroxide, potassium hydroxide, cesium hydroxide, magnesium hydroxide, calcium hydroxide, lithium hydride, sodium hydride, potassium hydride, sodium hydrogen carbonate, potassium hydrogen carbonate, trisodium phosphate, tripotassium phosphate, sodium methoxide, potassium methoxide, sodium ethoxide, potassium ethoxide, sodium tert-butoxide, potassium tert-butoxide, sodium tert-pentoxide, potassium tert-pentoxide, tetramethylammonium hydroxide, ethyltrimethylammonium hydroxide, tetraethylammonium hydroxide, tetrapropylammonium hydroxide, tetrabutylammonium hydroxide, tetraamylammonium hydroxide, tetrahexylammonium hydroxide, tetraheptylammonium hydroxide, choline hydroxide, benzyltrimethylammonium hydroxide, ammonia, n-butyllithium, triethylenediamine, 1,8-diazabicyclo[5.4.0]-7-undecene, N,N-diisopropylethylamine, 4-dimethylaminopyridine, and 1,1,3,3-tetramethylguanidine; and
a solvent is an organic solvent, water, or a combination thereof.

### [Invention 4]

The method according to Invention 3, wherein the base is one or more selected from sodium carbonate, potassium carbonate, sodium hydrogen carbonate, potassium hydrogen carbonate, sodium hydroxide, potassium hydroxide, trisodium phosphate, tripotassium phosphate, tetramethylammonium hydroxide, tetraethylammonium hydroxide, tetrapropylammonium hydroxide, tetrabutylammonium hydroxide, and benzyltrimethylammonium hydroxide.

### [Invention 5]

The method according to Invention 3, wherein the base is one or more selected from sodium carbonate, potassium carbonate, sodium hydroxide, potassium hydroxide, tetramethylammonium hydroxide, tetraethylammonium hydroxide, tetrapropylammonium hydroxide, tetrabutylammonium hydroxide, and benzyltrimethylammonium hydroxide.

### [Invention 6]

The method according to Invention 5, wherein the base is in the range of 0.001 to 3.0 mol per mol of the compound of formula (2).

### [Invention 7]

The method according to any one of Inventions 1 to 6, wherein a solvent is one or more selected from 2-propanol, acetone, methyl ethyl ketone, methyl isobutyl ketone, diisopropyl ketone, cyclohexanone, methanol, ethanol, tert-butyl alcohol, toluene, benzene, ethyl acetate, 1,4-dioxane, tetrahydrofuran, 2-methyltetrahydrofuran, methylene chloride, dichloroethane, chloroform, dimethyl sulfoxide, N,N-dimethylformamide, acetonitrile, water, and the compound (3).

### [Invention 8]

The method according to Invention 7, wherein the solvent is one or more selected from 2-propanol, acetone, methanol, ethanol, toluene, acetonitrile, tetrahydrofuran, and water.

### [Invention 9]

The method according to any one of Inventions 1 to 8, wherein a reaction temperature is 10°C to 110°C.

### [Invention 10]

The method according to any one of Inventions 1 to 9, wherein n is 1.

### [Invention 11]

The method according to Invention 1,
wherein all R¹ is hydrogen atoms;
R² is a hydrogen atom;
the base is one or more selected from sodium carbonate, potassium carbonate, sodium hydroxide, potassium hydroxide, tetramethyl ammonium hydroxide, tetraethylammonium hydroxide, tetrapropylammonium hydroxide, and tetrabutylammonium hydroxide;
a solvent is one or more selected from 2-propanol, acetone, methanol, ethanol, and water;
the base is in the range of 0.05 to 2.0 mol per mol of the compound of formula (2); and
a reaction temperature is 10°C to 90°C.

### [Invention 12]

The method according to any one of Inventions 1 to 11, wherein R³ is a formyl group.

### [Invention 13]

The method according to any one of Inventions 1 to 11, wherein R³ is a hydrogen atom.

### [Invention 14]

The method according to Invention 13, comprising:
- mixing a compound represented by formula (4): 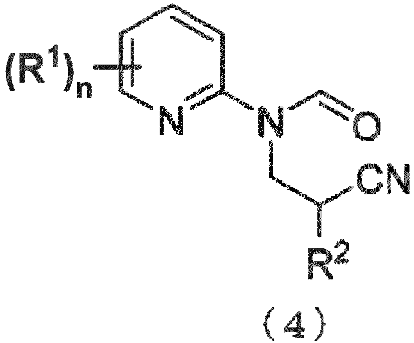 wherein
   each R¹ is as defined in formula (1);
   R² is as defined in formula (1); and
   n is as defined in formula (1)
with a base or an acid selected from hydrogen chloride, hydrochloric acid, hydrogen bromide, and hydrobromic acid.

### [Invention 15]

A compound represented by formula (4): 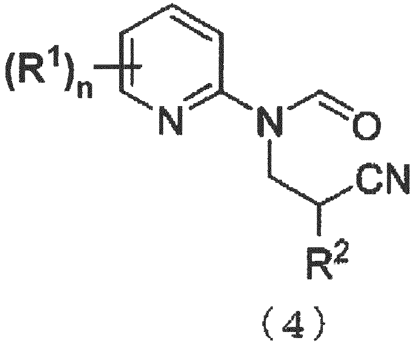 wherein
each R¹ independently represents a hydrogen atom, a halogen atom, a cyano group, a C1 to C4 alkyl group, a C1 to C4 haloalkyl group, a C1 to C4 alkoxy group, a C1 to C4 haloalkoxy group, a C1 to C4 alkylthio group, a C1 to C4 haloalkylthio group, a C1 to C4 alkylsulfonyl group, a C1 to C4 haloalkylsulfonyl group, a C1 to C4 alkylsulfone group, a C1 to C4 haloalkylsulfone group, or a pentafluorosulfanyl group;
R² is a hydrogen atom, a C1 to C4 alkyl group, a C1 to C4 haloalkyl group, or a C1 to C4 alkoxy group; and
n is 0 to 4.

### [Invention 16]

The compound according to Invention 15,
wherein each R¹ independently represents a hydrogen atom, a halogen atom, a C1 to C4 alkyl group, a C1 to C4 haloalkyl group, or a C1 to C4 haloalkoxy group; and
R² is a hydrogen atom or a C1 to C4 alkyl group.

### [Invention 17]

The compound according to Inventions 15 or 16, wherein n is 1.

### [Invention 18]

The compound according to Invention 14, wherein all R¹ is hydrogen atoms and R² is a hydrogen atom.

### Advantageous Effects of Invention

According to the present invention, 3-(pyridyl-2-amino)propionitrile and its analogues can be synthesized using industrially available starting materials and a target compound can be obtained with high yield, short step, and high substrate generality, and thus the producing method of the present invention is a method suitable for industrialization with less burden on the environment.

### Description of Embodiments

Hereinafter, the present invention will be described in detail.

The method for producing the compound of 3-(pyridyl-2-amino)propionitrile of the present invention and its analogues: formula (1) (hereinafter, which may be referred to as compound (1) and others are the same) comprises:
- mixing compound (2): 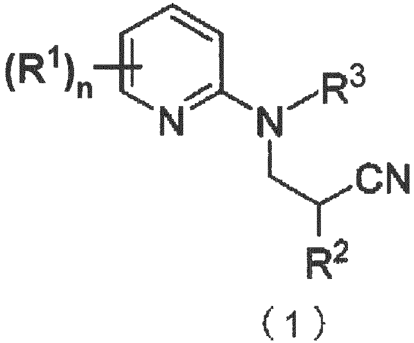 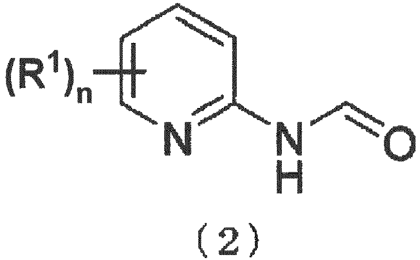 and the compound (3): 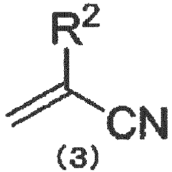 in the presence of a base to react the two (hereinafter, this step may be referred to as cyanoethylation step in some cases).

As the above base, one or more selected from lithium carbonate, sodium carbonate, potassium carbonate, cesium carbonate, lithium hydroxide, sodium hydroxide, potassium hydroxide, cesium hydroxide, magnesium hydroxide, calcium hydroxide, Lithium hydride, sodium hydride, potassium hydride, sodium hydrogen carbonate, potassium hydrogen carbonate, trisodium phosphate, tripotassium phosphate, sodium methoxide, potassium methoxide, sodium ethoxide, potassium ethoxide, sodium tert-butoxide, potassium tert-butoxide, sodium tert-pentoxide, potassium tert-pentoxide, tetramethylammonium hydroxide, ethyltrimethylammonium hydroxide, tetraethylammonium hydroxide, tetrapropylammonium hydroxide, tetrabutylammonium hydroxide, tetraamylammonium hydroxide, tetrahexylammonium hydroxide, tetraheptylammonium hydroxide, choline hydroxide, benzyltrimethylammonium hydroxide, ammonia, n-butyllithium, triethylenediamine, 1,8-diazabicyclo[5.4.0]-7-undecene, N,N-diisopropylethylamine, 4-dimethylaminopyridine, and 1,1,3,3-tetramethylguanidine can be used. In the present invention, sodium carbonate, potassium carbonate, sodium hydrogen carbonate, potassium hydrogen carbonate, sodium hydroxide, potassium hydroxide, trisodium phosphate, tripotassium phosphate, tetramethylammonium hydroxide, tetraethylammonium hydroxide, tetrapropylammonium hydroxide, tetrabutylammonium hydroxide, and benzyltrimethylammonium hydroxide are preferable; and sodium carbonate, potassium carbonate, sodium hydroxide, potassium hydroxide, tetramethylammonium hydroxide, tetraethylammonium hydroxide, tetrapropylammonium hydroxide, tetrabutylammonium hydroxide, and benzyltrimethylammonium hydroxide are more preferable. The amount of the base is in the range of 0.001 to 3.0 mol and more preferably in the range of 0.05 to 2.0 mol per mol of the compound (2). The base can be changed during the reaction.

The mixing order of the compound (2), the compound (3), and the base is not particularly limited, and the compound (3) and a base may be mixed with the compound (2), the compound (2) and a base may be mixed with the compound (3), and preferably the compound (2) and a base are mixed and then the compound (3) is mixed with the obtained mixture. The above mixing may be performed in a solvent.

In the present reaction, a solvent may be used. Examples of the solvent include 2-propanol, acetone, methyl ethyl ketone, methyl isobutyl ketone, diisopropyl ketone, cyclohexanone, methanol, ethanol, tert-butyl alcohol, toluene, benzene, ethyl acetate, 1,4-dioxane, tetrahydrofuran, 2-methyltetrahydrofuran, methylene chloride, dichloroethane, chloroform, acetonitrile, dimethyl sulfoxide, N,N-dimethylformamide, and water; and preferably 2-propanol, acetone, methanol, ethanol, toluene, acetonitrile, tetrahydrofuran, and water. The solvent can be changed during the reaction. An excess amount of the compound (2) or the compound (3) may be used to serve as a solvent.

The temperature in the cyanoethylation step is, for example, in the range of 0 to 120°C and preferably 40 to 100°C. The period of the cyanoethylation step is, for example, in the range of 5 minutes to 48 hours and preferably 30 minutes to 5 hours.

The reaction mixture (reaction solution or the like) obtained in the cyanoethylation step may be subjected to post treatment such as extraction with an organic solvent or the like after adding water or the like or washing with water and may be subjected to purification such as filtration, crystallization, extraction, distillation, an adsorption method such as activated carbon and silica alumina, and a chromatographic method such as silica gel column chromatography. The organic solvent is not particularly limited and examples thereof include toluene, n-hexane, n-pentane, n-heptane, ethyl acetate, methylene chloride, methanol, ethanol, and diethyl ether. It is possible to take out as a salt using an acid. Examples of the acid include hydrogen chloride, hydrochloric acid, hydrogen bromide, hydrobromic acid, sulfuric acid, nitric acid, picric acid, and the like.

Examples of R¹ in the compounds (1) and (2) include a hydrogen atom, a C1 to C4 alkyl group, a C1 to C4 haloalkyl group, a C1 to C4 alkoxy group, a halogen atom, a cyano group, a C1 to C4 alkylthio group, a C1 to C4 haloalkylthio group, a C1 to C4 alkylsulfonyl group, a C1 to C4 haloalkylsulfonyl group, a C1 to C4 alkylsulfone group, a C1 to C4 haloalkylsulfone group, and a pentafluorosulfanyl group; preferably a hydrogen atom, a fluorine atom, a chlorine atom, a bromine atom, an iodine atom, a methyl group, an ethyl group, a methoxy group, a trifluoromethyl group, and a trifluoromethoxy group; and more preferably a hydrogen atom, a fluorine atom, a chlorine atom, a bromine atom, a methyl group, and a trifluoromethyl group. Each R¹ is independently selected.

Examples of R² in the compounds (1) and (3) include a hydrogen atom, a C1 to C4 alkyl group, a C1 to C4 haloalkyl group, and a C1 to C4 alkoxy group; preferably a methyl group, an ethyl group, a trifluoromethyl group, a methyloxy group, and an ethyloxy group; and more preferably a hydrogen atom and a methyl group.

R³ in the compound (1) is a hydrogen atom or a formyl group.

n in the compounds (1) and (2) is 0 to 4.

Examples of the compound (1) include 3-(pyridyl-2-amino)propionitrile, 3 -((4-trifluoromethylpyridyl)-2-amino)propionitrile, 3-((5-bromopyridyl)-2-amino) propionitrile, 3-((6-methylpyridyl)-2-amino)propionitrile, 2-methyl-3-(pyridyl-2-amino)propionitrile, and 3-((3-methylpyridyl)-2-amino)propionitrile. The present invention is not limited only to the synthesis of these compounds and includes all combinations with respect to R¹, R², R³, n, and substitution positions as defined above.

The reaction scheme of the present invention is shown below. The compound (1) can be obtained by reacting the compound (2) and the compound (3). Under a predetermined condition, the compound (1) in which R³ is a hydrogen atom (which may be referred to as the compound (1')) is obtained. Under a predetermined condition, the compound (1) in which R³ is a formyl group, that is, the compound (4) can be selectively obtained. It is also possible to synthesize the compound (1') from the compound (4). The compound (4) was confirmed by gas chromatography analysis of Example 1-1, and the compound (4) was the main product at the initial stage of reaction. In Example 1-2 and Step 1, a method for selectively obtaining the compound (4) is shown. The cyano group is widely known to be hydrolyzed under acidic and basic conditions, but in the present invention, only deprotection (deformylation) reaction proceeds promptly by using appropriate conditions.

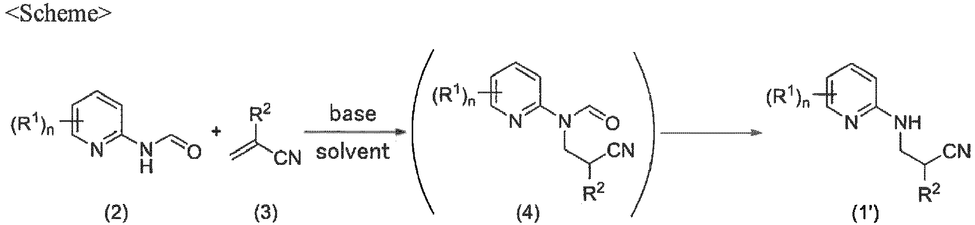

The predetermined condition for obtaining the compound (4) is, for example, a method in which the compounds (2) and (3) are reacted while monitoring with gas chromatography or the like and the reaction is terminated at an appropriate timing. Properly selecting bases and solvents also can provide the compound (4). For example, using an inorganic base such as potassium carbonate, and acetone or acetonitrile as a solvent can provide the compound (4). The compound (4) can be obtained by using an organic base such as tetramethylammonium hydroxide, and 2-propanol, acetone, methanol, ethanol, tert-butyl alcohol, toluene, benzene, ethyl acetate, 1,4-dioxane, methylene chloride, or acetonitrile as a solvent. A specific example of synthesis is described in Example 1-1, Step 1 of Example 1-2, and the first row of Example 1-3. For the deprotection reaction using an acid, the desired product can be obtained by neutralization with a base followed by extraction or can be obtained as a salt by evaporation of a solvent.

An example of the compound (4) includes a compound in which each R¹ independently represents a hydrogen atom, a halogen atom, a cyano group, a C1 to C4 alkyl group, a C1 to C4 haloalkyl group, a C1 to C4 alkoxy group, a C1 to C4 haloalkoxy group, a C1 to C4 alkylthio group, a C1 to C4 haloalkylthio group, a C1 to C4 alkylsulfonyl group, a C1 to C4 haloalkylsulfonyl group, a C1 to C4 alkylsulfone group, a C1 to C4 haloalkylsulfone group, or a pentafluorosulfanyl group and R² represents a hydrogen atom, a C1 to C4 alkyl group, a C1 to C4 haloalkyl group, or a C1 to C4 alkoxy group. A preferable example of the compound (4) include a compound in which each R¹ independently represents a hydrogen atom, a halogen atom, a C1 to C4 alkyl group, a C1 to C4 haloalkyl group, or a C1 to C4 haloalkoxy group, and R² is a hydrogen atom or a C1 to C4 alkyl group. More preferable examples of the compound (4) include compound (4') in which all R¹ represents a hydrogen atom and R² represents a hydrogen atom.

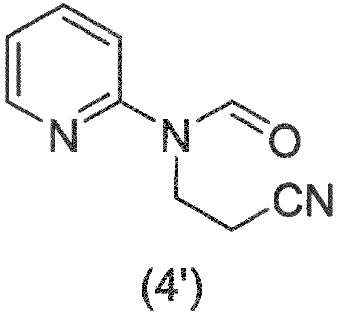

The compound (4) is a useful compound as a synthetic intermediate of the compound (1'), but additionally has mite insecticidal activity and is a compound useful as an agricultural chemical. The compound (1') has insecticidal activity against rice brown planthopper and is also useful as an agricultural chemical. Specific test examples are described in biological test examples.

Formamide can be deprotected by using an acid or a base (Greene's Protective Groups in Organic Synthesis Fifth Edition, Peter G. M. Wuts, John Wiley & Sons Inc, 2014). In this reaction, deprotection can be performed under the condition from room temperature to reflux temperature. Preferable examples of the acid include hydrochloric acid and hydrobromic acid, and preferable examples of the base include sodium carbonate, potassium carbonate, sodium hydroxide, and potassium hydroxide. The reaction temperature is 0°C to 80°C and more preferably 20°C to 60°C. When an acid is used, the target product can be taken out by neutralization or as a salt. When a base is used, neutralization is performed and the target product can be taken out by extraction or the like. The reaction period is 5 minutes to 12 hours and more preferably 1 hour to 6 hours.

### Examples

Hereinafter, the present invention will be described in more detail with reference to Examples, but the present invention is not limited to Examples.

### <Example 1-1>

### Synthesis of 3-(pyridyl-2-amino)propionitrile

244 mg (2.0 mmol) of N-2-pyridylformamide, 304 mg (2.2 mmol) of potassium carbonate, and 20 mL of 2-propanol were placed in a vessel, and 0.39 mL (6.0 mmol) of acrylonitrile was added dropwise thereto at room temperature. After that, the mixture was heated to 75°C and stirred for 1 hour. After 1 hour, the gas chromatographic area ratio of (3-(pyridyl-2-amino)propionitrile to N-(2-cyanoethyl)-N-(2-pyridyl)formamide) was 24:76. After 2 hours, the area ratio of (3-(pyridyl-2-amino)propionitrile to N-(2-cyanoethyl)-N-(2-pyridyl)formamide) was 53:47. The mixture was further stirred at 75°C for 3 hours. The mixture was then cooled to room temperature, water was added, followed by extracting with ethyl acetate and washing with brine, and the solvent was evaporated under reduced pressure. 401 mg of the target product (purity: 70.3%, pure yield: 95.8%) was obtained.
¹H NMR (CDCl₃) δ = 8.09 (1H, dd, J = 4.8, 0.9Hz), 7.35-7.47 (1H, m), 6.62 (1H, ddd, J = 6.3, 5.1, 0.9Hz), 6.44 (1H, d, J = 8.4Hz), 4.73 (1H, broad), 3.70 (2H, q, J = 6.3Hz), 2.74 (2H, t, J = 6.3Hz)

### <Example 1-2>

### Synthesis of 3-(pyridyl-2-amino)propionitrile

244 mg (2.0 mmol) of N-2-pyridylformamide, 82.8 mg (0.6 mmol) of potassium carbonate, and 20 mL of 2-propanol were placed in a vessel, and 0.39 mL (6.0 mmol) of acrylonitrile was added dropwise thereto at room temperature, then the mixture was heated to reflux for 5 hours. The mixture was then cooled to room temperature, water was added, followed by extracting with ethyl acetate and washing with brine, and the solvent was evaporated under reduced pressure. 465 mg of the target product (purity: 62.3%, pure yield: 98%) was obtained.

### Synthesis of 3-(pyridyl-2-amino)propionitrile (alternative method 1)

### <Example 2, Step 1>

### Synthesis of N-(2-cyanoethyl)-N-(2-pyridyl)formamide

244 mg (2.0 mmol) of N-2-pyridylformamide, 304 mg (2.2 mmol) of potassium carbonate, and 20 mL of acetone were placed in a vessel, and 0.39 mL (6.0 mmol) of acrylonitrile was added dropwise thereto at room temperature. After that, the mixture was heated to reflux and stirred for 2 hours. After cooling to room temperature, the reaction solution was filtered, the solvent was evaporated under reduced pressure, and the reaction solution was purified by silica gel column chromatography. 329 mg of the target product (yield: 94%, white solid) was obtained.

In this reaction, even when 20 mL of acetonitrile was used instead of 20 mL of acetone, 348 mg of the target product (yield: 99%) was obtained.
¹H NMR (CDCl₃) δ = 9.01 (1H, s), 8.44-8.46 (1H, m), 7.73-7.79 (1H, m), 7.14-7.21 (2H, m), 4.31 (2H, t, J = 7.0Hz), 2.82 (2H, t, J = 7.0Hz)
GCMS; 174.91 (Exact Mass: 175.07), mp; 43-46°C

### <Example 2, Step 2>

350 mg (2.0 mmol) of N-(2-cyanoethyl)-N-(2-pyridyl)formamide, 304 mg (2.2 mmol) of potassium carbonate, and 20 mL of 2-propanol were placed in a vessel and heated to 75°C. After 5 hours, the mixture was cooled to room temperature, water was added, followed by extracting with ethyl acetate and washing with brine, and the solvent was evaporated under reduced pressure. 289 mg (yield: 98%) of the target product was obtained.

In this reaction, even when the reaction mixture was heated to reflux for 1 hour using 20 mL of methanol instead of 20 mL of 2-propanol, 260 mg (yield: 88%) of the target product was obtained.

### Synthesis of 3-(pyridyl-2-amino)propionitrile (alternative method 2)

### <Example 3>

244 mg (2.0 mmol) of N-2-pyridylformamide, 27.6 mg (0.2 mmol) of potassium carbonate, and 2.0 mL of acetone were placed in a vessel, and 0.195 mL (3.0 mmol) of acrylonitrile was added dropwise thereto at room temperature. After that, the mixture was heated to reflux and stirred for 2 hours. After cooling to room temperature, the solvent and acrylonitrile were evaporated under reduced pressure. N-(2-cyanoethyl)-N-(2-pyridyl)formamide was obtained as a crude product.

88 mg (2.2 mmol) of sodium hydroxide and 10 mL of tap water were added thereto, and the mixture was heated to 40°C and stirred for 1 hour. After that, the mixture was cooled to room temperature, followed by extracting with toluene and washing with brine, and the solvent was evaporated under reduced pressure. 265 mg (yield: 90%) of the target product was obtained.

### Synthesis of 3-(pyridyl-2-amino) propionitrile (alternative method 3)

### <Example 4>

244 mg (2.0 mmol) of N-2-pyridylformamide, 2.0 mL of acetone, and 0.195 mL (3.0 mmol) of acrylonitrile were placed in a vessel, and 0.7 mL of a 10% tetrabutylammonium hydroxide aqueous solution was added dropwise thereto at room temperature. After that, the mixture was heated to 60°C and stirred for 3 hours. After cooling to room temperature, the solvent and acrylonitrile were evaporated under reduced pressure. As a crude product, N-(2-cyanoethyl)-N-(2-pyridyl)formamide was obtained.

88 mg (2.2 mmol) of sodium hydroxide and 10 mL of tap water were added thereto, and the mixture was heated to 40°C and stirred for 1 hour. The mixture was then cooled to room temperature, extracted with toluene, washed with water and brine, and the solvent was evaporated under reduced pressure. 238 mg (yield: 81%) of the target product was obtained.

### Synthesis of 3-(pyridyl-2-amino)propionitrile (alternative method 4)

### <Example 5>

156 mg (0.89 mmol) of N-(2-cyanoethyl)-N-(2-pyridyl)formamide and 10 mL of tetrahydrofuran were placed in a vessel, 2.0 mL of 6N-HCl was added, and the mixture was stirred at room temperature for 5 hours. After confirming the disappearance of the starting material by TLC, the mixture was quenched using aqueous sodium hydrogen carbonate solution, followed by extracting with ethyl acetate and washing with brine, and the solvent was evaporated under reduced pressure. 130 mg (yield: 99%) of the target product was obtained.

### <Example 6>

### Synthesis of 3-((4-trifluoromethylpyridyl)-2-amino)propionitrile

A reaction was performed in the same manner as in Example 1-1 except that N-2-pyridylformamide was changed to N-((4-trifluorometliyl)2-pyridyl)formamide (2.0 mmol). The crude product was purified by column chromatography to obtain 333 mg of the target product (yield: 74%, white solid).
¹H NMR (CDCl₃) δ = (1H, d, J = 5.1Hz), 6.81 (1H, d, J = 5.1Hz), 6.64 (1H, s), 5.00 (1H, broad), 3.75 (2H, q, J = 6.3Hz), 2.76 (2H, t, J = 6.2Hz)
mp; 59-62°C

### <Example 7>

### Synthesis of 3-((5-bromopyridyl)-2-amino)propionitrile

A reaction was performed in the same manner as in Example 1-1 except that N-2-pyridylformamide was changed to N-(5-bromo-2-pyridyl)formamide (2.0 mmol). The crude product was purified by column chromatography to obtain 304 mg of the target product (yield: 71%, white solid).
¹H NMR (CDCl₃) δ = (1H, d, J = 2.4Hz), 7.48 (1H, dd, J = 8.7, 2.7Hz), 6.37 (1H, d, J = 9.0Hz), 4.79 (1H, broad), 3.68 (2H, q, J = 6.3Hz), 2.73 (2H, t, J = 6.3Hz)
mp; 72-74°C

### <Example 8>

### Synthesis of 3-((6-Methylpyridyl)-2-amino)propionitrile

A reaction was performed in the same manner as in Example 1-1 except that N-2-pyridylformamide was changed to N-(6-Methyl-2-pyridyl)formamide (2.0 mmol). The crude product was purified by column chromatography to obtain 238 mg of the target product (yield: 74%, clear liquid).
¹H NMR (CDCl₃) NMR δ = (1H, t, J = 7.8Hz), 6.49 (1H, d, J = 7.2Hz), 6.23 (1H, d, J = 8.1Hz), 4.67 (1H, broad), 3.68 (2H, q, J = 6.4Hz), 2.74 (2H, t, J = 6.4Hz), 2.37 (3H, s)

### <Example 9>

### Synthesis of 2-methyl-3-(pyridyl-2-amino)propionitrile

A reaction was performed in the same manner as in Example 1-1 except that acrylonitrile was changed to methacrylonitrile (6.0 mmol). The crude product was purified by column chromatography to obtain 28.9 mg of the target product (yield: 9%, clear liquid).
¹H NMR (CDCl₃) δ = 8.08 (1H, dd, J = 5.1, 1.2Hz), 7.37-7.43 (1H, m), 6.58-6.63 (1H, m), 6.44 (1H, d, J = 8.4Hz), 4.87 (1H, broad), 3.65-3.75 (1H, m), 3.38-3.48 (1H, m), 3.10-3.22 (1H, m), 1.35 (3H, d, J = 7.2Hz)

### <Example 10>

### Synthesis of 3-(pyridyl-2-amino)propionitrile hydrochloride

244 mg (2.0 mmol) of N-2-pyridylformamide, 304 mg (2.2 mmol) of potassium carbonate, and 20 mL of 2-propanol were placed in a vessel, and 0.39 mL (6.0 mmol) of acrylonitrile was dropped thereto at room temperature. After that, the mixture was heated to 80°C and stirred for 5 hours. The mixture was then cooled to room temperature, water was added, followed by extracting with ethyl acetate and washing with brine, and the solvent was evaporated under reduced pressure to obtain the crude product which is a mixture of N-(2-cyanoethyl)-N-(2-pyridyl)formamide and the final product.

The obtained crude product was dissolved in 10 mL of diethyl ether, 5.0 mL of hydrogen chloride (diethyl ether solution, 1 mol/L) was added, and the mixture was stirred at room temperature for 1 hour. The precipitated white solid was filtered off, and the filtered solid was dried at 60°C with warm air. 340 mg (yield: 92%) of the target product was obtained.
¹H NMR (d6 DMSO) δ = 14.37 (1H, broad), 9.22 (1H, broad), 7.91-7.96 (2H, m), 7.17 (1H, d, J = 9.0Hz), 6.92 (1H, t, J = 6.6Hz), 3.76 (2H, q, J = 6.0Hz), 2.89 (2H, t, J = 6.6Hz) mp; 125-128°C

### <Reference example 1>

### Synthesis of 2-hydroxy-4-oxo-3-(3-trifluoromethylphenyl)-1-(2-cyanoethyl)-4H-pyrido[1,2-a]pyrimidinium inner salt

3-Pyridyl-2-aminopropionitrile (synthesized in Example 1-1 and unpurified, 147 mg) and bis(2,4,6-trichlorophenyl)2-(3-trifluoromethylphenyl) propanedioate (607 mg, 1.0 mmol) were added in toluene (10 mL). By heating the reaction mixture at 110°C for 2 hours, a yellow solid was precipitated out of the solution. The reaction mixture was cooled to room temperature and filtered, and the filtered crystal was dried at 60°C with warm air. 227 mg of the target product (yield: 63%, yellow solid, purity: 90%, HPLC area%) was obtained.
¹H NMR (d6 DMSO) δ = 9.60 (1H, ddd, J = 6.9, 1.8, 0.6Hz), 8.28-8.19 (1H, m), 8.15-8.10 (1H, m), 8.05-7.97 (1H, m), 7.75 (1H, d, J = 8.7Hz), 7.56-7.49 (2H, m), 7.47 (1H, td, J = 7.2, 0.9Hz), 4.66 (2H, t, J = 6.3Hz), 3.02 (2H, t, J = 6.6Hz)

### <Reference Example 2>

### Synthesis of 2-hydroxy-4-oxo-3-(3-trifluoromethylphenyl)-1-(2-cyano-ethyl)-4H-pyrido[1,2-a]pyrimidinium inner salt

3-Pyridyl-2-aminopropionitrile (synthesized in Example 3 and unpurified, 147 mg) and bis(2,4,6-trichlorophenyl)2-(3-trifluoromethylphenyl)propanedioate (607 mg, 1.0 mmol) were added in toluene (10 mL). By heating the reaction mixture at 110°C for 2 hours, a yellow solid was precipitated out of the solution. The reaction mixture was cooled to room temperature and filtered, and the filtered crystal was dried at 60°C with warm air. 267 mg of the target product (yield: 74%, yellow solid, purity: 98.5%, HPLC area%) was obtained.

### Biological test example

### Biological test example 1: Pest control test (leaf soaking treatment) to twospotted spider mite (Tetranychus urticae)

A bean leaf was cut to a diameter of 3.5 cm and placed on absorbent cotton moistened with water. Five adult females of the twospotted spider mite were released on the bean leaf and laid eggs for 24 hours, and then the adults were removed. This bean leaf was soaked for about 30 seconds in 20 mL of the dilution solution of the test compound diluted to 800 ppm. After air-drying, the bean leaf was put in a plastic cup together with absorbent cotton, covered, and kept in a constant temperature room at 25°C. In seven days after treatment, life and death were observed and the ovicidal rate was calculated.

### N-(2-cyanoethyl)-N-(2-pyridyl)formamide (that is, compound (4')), used as a test compound, showed 100% of the pest control effect

### Biological test example 2: Pest control test (stem and leaf soaking treatment) to rice brown planthopper (Nilaparvata lugens)

Ten rice seedlings were soaked in 20 mL of the test compound solution diluted to 800 ppm and air-dried. After air drying, the rice seedings were hold in a glass cylinder (inner diameter 4.5 cm x 14 cm) with urethane and stood on the plastic cup in which 40 mL of water was put. The 3-year-old larva of rice brown planthopper was released there, covered with medicine package, and kept in a constant temperature room at 25°C. In five days after treatment, life and death were observed and the mortality was calculated.

3-(Pyridyl-2-amino)propionitrile (that is, compound (1')), used as a test compound, showed 50% of the control effect.

### Industrial Applicability

3-(Pyridyl-2-amino)propionitrile and its analogues are used as synthetic intermediates for pharmaceuticals, pesticides, and the like, and the producing method of the present invention can synthesize 3-(pyridyl-2-amino)propionitrile and its analogues efficiently and in high yield. N-(2-cyanoethyl)-N-(2-pyridyl)formamide and its analogues are important as reaction intermediates of the present invention, and through this compound, 3-(pyridyl-2-amino) propionitrile and its analogues can be synthesized efficiently and in high yield. 3-(pyridyl-2-amino)propionitrile and its analogues, and N-(2-cyanoethyl)-N-(2-pyridyl)formamide and its analogues, have insecticidal and ovicidal activity and are useful as agricultural chemicals.

## Claims

1. A method for producing a compound represented by formula (1): 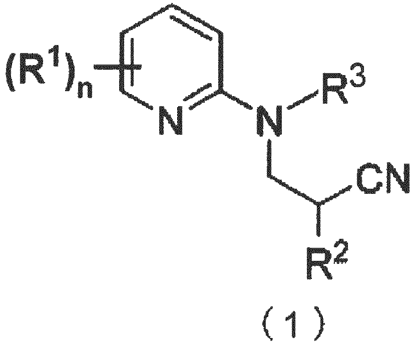
wherein each R¹ independently represents a hydrogen atom, a halogen atom, a cyano group, a C1 to C4 alkyl group, a C1 to C4 haloalkyl group, a C1 to C4 alkoxy group, a C1 to C4 haloalkoxy group, a C1 to C4 alkylthio group, a C1 to C4 haloalkylthio group, a C1 to C4 alkylsulfonyl group, a C1 to C4 haloalkylsulfonyl group, a C1 to C4 alkylsulfone group, a C1 to C4 haloalkylsulfone group, or a pentafluorosulfanyl group;
R² is a hydrogen atom, a C1 to C4 alkyl group, a C1 to C4 haloalkyl group, or a C1 to C4 alkoxy group;
R³ is a hydrogen atom or a formyl group; and
n is 0 to 4,
the method comprising:
- mixing a compound represented by formula (2): 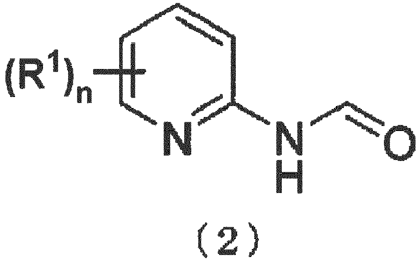 wherein each R¹ is as defined in formula (1), and
a compound represented by formula (3): 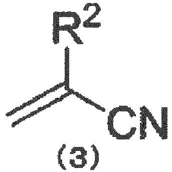 wherein R² is as defined in formula (1),
in the presence of a base to react the two.

2. The method according to claim 1, wherein each R¹ independently represents a hydrogen atom, a halogen atom, a C1 to C4 alkyl group, a C1 to C4 haloalkyl group, or a C1 to C4 haloalkoxy group; and
R² is a hydrogen atom or a C1 to C4 alkyl group.

3. The method according to claim 1 or 2, wherein the base is one or more selected from lithium carbonate, sodium carbonate, potassium carbonate, cesium carbonate, lithium hydroxide, sodium hydroxide, potassium hydroxide, cesium hydroxide, magnesium hydroxide, calcium hydroxide, lithium hydride, sodium hydride, potassium hydride, sodium hydrogen carbonate, potassium hydrogen carbonate, trisodium phosphate, tripotassium phosphate, sodium methoxide, potassium methoxide, sodium ethoxide, potassium ethoxide, sodium tert-butoxide, potassium tert-butoxide, sodium tert-pentoxide, potassium tert-pentoxide, tetramethylammonium hydroxide, ethyltrimethylammonium hydroxide, tetraethylammonium hydroxide, tetrapropylammonium hydroxide, tetrabutylammonium hydroxide, tetraamylammonium hydroxide, tetrahexylammonium hydroxide, tetraheptylammonium hydroxide, choline hydroxide, benzyltrimethylammonium hydroxide, ammonia, n-butyllithium, triethylenediamine, 1,8-diazabicyclo[5.4.0]-7-undecene, N,N-diisopropylethylamine, 4-dimethylaminopyridine, and 1,1,3,3-tetramethylguanidine; and
a solvent is an organic solvent, water, or a combination thereof.

4. The method according to claim 3, wherein the base is one or more selected from sodium carbonate, potassium carbonate, sodium hydrogen carbonate, potassium hydrogen carbonate, sodium hydroxide, potassium hydroxide, trisodium phosphate, tripotassium phosphate, tetramethylammonium hydroxide, tetraethylammonium hydroxide, tetrapropylammonium hydroxide, tetrabutylammonium hydroxide, and benzyltrimethylammonium hydroxide.

5. The method according to claim 3, wherein the base is one or more selected from sodium carbonate, potassium carbonate, sodium hydroxide, potassium hydroxide, tetramethylammonium hydroxide, tetraethylammonium hydroxide, tetrapropylammonium hydroxide, tetrabutylammonium hydroxide, and benzyltrimethylammonium hydroxide.

6. The method according to claim 5, wherein the base is in the range of 0.001 to 3.0 mol per mol of the compound of formula (2).

7. The method according to any one of claims 1 to 6, wherein a solvent is one or more selected from 2-propanol, acetone, methyl ethyl ketone, methyl isobutyl ketone, diisopropyl ketone, cyclohexanone, methanol, ethanol, tert-butyl alcohol, toluene, benzene, ethyl acetate, 1,4-dioxane, tetrahydrofuran, 2-methyltetrahydrofuran, methylene chloride, dichloroethane, chloroform, dimethyl sulfoxide, N,N-dimethylformamide, acetonitrile, water, and the compound (3).

8. The method according to claim 7, wherein the solvent is one or more selected from 2-propanol, acetone, methanol, ethanol, toluene, acetonitrile, tetrahydrofuran, and water.

9. The method according to any one of claims 1 to 8, wherein a reaction temperature is 10°C to 110°C.

10. The method according to any one of claims 1 to 9, wherein n is 1.

11. The method according to claim 1,
wherein all R¹ is hydrogen atoms;
R² is a hydrogen atom;
the base is one or more selected from sodium carbonate, potassium carbonate, sodium hydroxide, potassium hydroxide, tetramethylammonium hydroxide, tetraethylammonium hydroxide, tetrapropylammonium hydroxide, and tetrabutylammonium hydroxide;
the solvent is one or more selected from 2-propanol, acetone, methanol, ethanol, and water;
the base is in the range of 0.05 to 2.0 mol per mol of the compound of formula (2); and
a reaction temperature is 10°C to 90°C.

12. The method according to any one of claims 1 to 11, wherein R³ is a formyl group.

13. The method according to any one of claims 1 to 11, wherein R³ is a hydrogen atom.

14. The method according to claim 13, comprising the step of mixing a compound represented by formula (4): 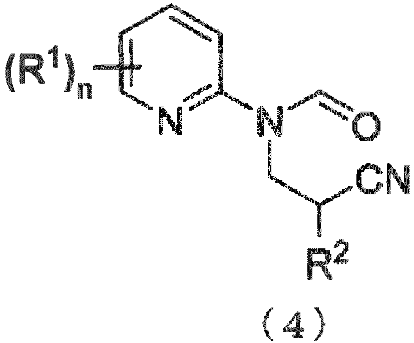 wherein
each R¹ is as defined in formula (1);
R² is as defined in formula (1); and
n is as defined in formula (1)
with a base or an acid selected from hydrogen chloride, hydrochloric acid, hydrogen bromide, and hydrobromic acid.

15. A compound represented by formula (4): 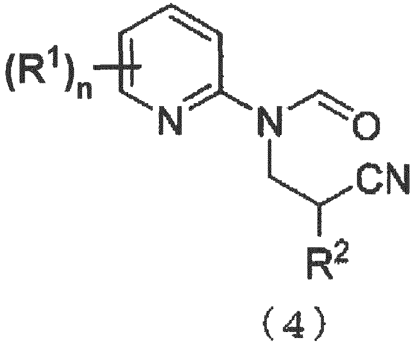 wherein
each R¹ independently represents a hydrogen atom, a halogen atom, a cyano group, a C1 to C4 alkyl group, a C1 to C4 haloalkyl group, a C1 to C4 alkoxy group, a C1 to C4 haloalkoxy group, a C1 to C4 alkylthio group, a C1 to C4 haloalkylthio group, a C1 to C4 alkylsulfonyl group, a C1 to C4 haloalkylsulfonyl group, a C1 to C4 alkylsulfone group, a C1 to C4 haloalkylsulfone group, or a pentafluorosulfanyl group;
R² is a hydrogen atom, a C1 to C4 alkyl group, a C1 to C4 haloalkyl group, or a C1 to C4 alkoxy group; and
n is 0 to 4.

16. The compound according to claim 15, wherein each R¹ independently represents a hydrogen atom, a halogen atom, a C1 to C4 alkyl group, a C1 to C4 haloalkyl group, or a C1 to C4 haloalkoxy group; and
R² is a hydrogen atom or a C1 to C4 alkyl group.

17. The compound according to claim 15 or 16, wherein n is 1.

18. The compound according to claim 14, wherein all R¹ is hydrogen atoms and R² is a hydrogen atom.
